(19) Europäisches Patentamt
European Patent Office
Office européen des brevets

(11) **EP 4 101 377 B1**

(12) **EUROPEAN PATENT SPECIFICATION**

(45) Date of publication and mention
of the grant of the patent:
**17.09.2025 Bulletin 2025/38**

(21) Application number: **21305782.1**

(22) Date of filing: **09.06.2021**

(51) International Patent Classification (IPC):
**A61B 5/113** *(2006.01)*     **A61B 5/00** *(2006.01)*

(52) Cooperative Patent Classification (CPC):
**A61B 5/113; A61B 5/6803;** A61B 2560/0209

(54) **METHOD FOR DETERMINING CARDIAC OR RESPIRATORY ACTIVITY OF A SUBJECT AND ASSOCIATED SYSTEM**

VERFAHREN ZUR BESTIMMUNG DER HERZ- ODER ATMUNGSAKTIVITÄT EINES PATIENTEN UND ZUGEHÖRIGES SYSTEM

PROCÉDÉ DE DÉTERMINATION DE L'ACTIVITÉ CARDIAQUE OU RESPIRATOIRE D'UN SUJET ET SYSTÈME ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.12.2022 Bulletin 2022/50**

(73) Proprietor: **ESSILOR INTERNATIONAL**
**94220 Charenton-Le-Pont (FR)**

(72) Inventors:
• **GIL, Paul**
**80000 Amiens (FR)**
• **AMIR, Bruno**
**94100 Saint Maur des Fosses (FR)**
• **SAHLER, Jean**
**92160 Antony (FR)**

(74) Representative: **Jacobacci Coralis Harle**
**32, rue de l'Arcade**
**75008 Paris (FR)**

(56) References cited:
US-A1- 2015 265 161     US-A1- 2017 220 772
US-A1- 2018 214 030     US-A1- 2019 231 197
US-A1- 2021 063 434

• HERNANDEZ JAVIER ET AL: "Wearable Motion-Based Heart Rate at Rest: A Workplace Evaluation", IEEE JOURNAL OF BIOMEDICAL AND HEALTH INFORMATICS, IEEE, PISCATAWAY, NJ, USA, vol. 23, no. 5, 1 September 2019 (2019-09-01), pages 1920 - 1927, XP011743829, ISSN: 2168-2194, [retrieved on 20190903], DOI: 10.1109/JBHI.2018.2877484
• HERNANDEZ J. ET AL: "Cardiac and Respiratory Parameter Estimation Using Head-mounted Motion-sensitive Sensors", vol. 1, no. 1, 1 May 2015 (2015-05-01), pages e2, XP055856619, Retrieved from the Internet <URL:https://citeseerx.ist.psu.edu/viewdoc/download?doi=10.1.1.694.196&rep=rep1&type=pdf> DOI: 10.4108/phat.1.1.e2

# EP 4 101 377 B1

**Description**

TECHNICAL FIELD OF THE INVENTION

The present invention relates to the field of health monitoring.

The present invention relates in particular to a method for determining cardiac or respiratory activity of a subject, and to an associated system.

BACKGROUND INFORMATION AND PRIOR ART

Cardiac and respiratory activity information are great indicators for health and wellness. Such indicators may be used for people's disease detection, self-health monitoring, telemedicine, stress level monitoring, driving level of attention monitoring, or for other applications such as sport coaching, video games.

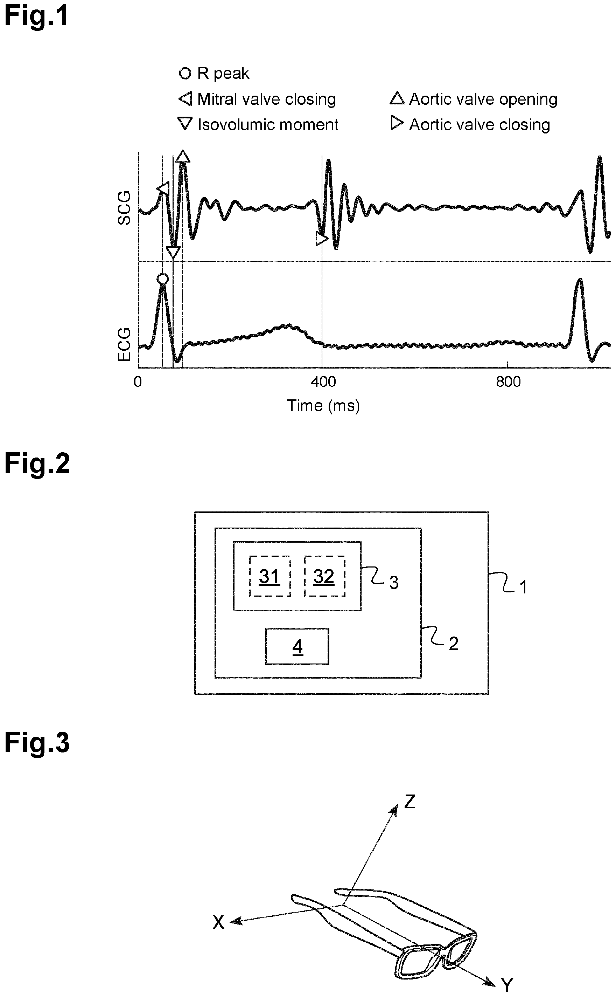

Seismocardiography (SCG) is a technique of measuring the vibrations of the chest produced by the beating heart, which uses an accelerometer to record those vibrations. In comparison with an electrocardiogram, SCG provides with detailed information of cardiac events in each heartbeat. Figure 1 shows a comparison between a SCG measurement and a corresponding electrocardiogram. It can be seen that the SCG measurement provides more accurate details of the heart activity.

Gyrocardiography (GCG) is a more recent, non invasive technique for acquisition and analysis of changes in angular velocity of the chest associated with cardiac activity.

The combination of both SCG and GCG techniques allows obtaining measurements with increased accuracy.

Usually, the SCG/GCG measurement sensor is an inertial measurement unit consisting of a MEMS (Microelectromechanical systems) accelerometer and gyroscope that is placed on the sternum, close to the heart. Subjects are positioned in a supine position, or the measurement sensor is attached to the skin.

The obtained measurements may also be used to estimate the respiratory rate, volume and phases of subjects, as described in the international application WO 2020/037391. US 2015/265161 A1 describes methods and apparatus for physiological parameter estimation.

Optical heart beat sensors as in smart watches use a technology called photoplethysmography. This technology uses green LED lights paired with light-sensitive photodiodes to detect the amount of blood flowing through the wrist at any given moment. The heart beats correspond to greater green light absorption of the blood flow in the wrist. However, this technology is power consuming, requires space and is difficult to integrate mechanically. Moreover, sensors for photoplethysmography are specifically dedicated to measure cardiac activity.

Therefore, no solution currently exists for low-power, space-reduced continuous monitoring of cardiac and respiratory activity of a subject.

SUMMARY OF THE INVENTION

In this context, the present invention proposes a method for determining cardiac or respiratory activity of a subject, as claimed in claim 1.

Therefore, the method according to the invention aims at providing with a solution to measure the cardiac and respiratory activity of a subject in a non-constraint, permanent or semi-permanent manner.

In addition, the invention presents the non-obvious advantage that no sensor has to be placed in direct contact with the subject's body and close to the heart, as compared to classical SCG/GCG measurements. In the invention, no tight mechanical interface is needed between the subject's body and the accessory. The invention thus defeats the prior art prejudice according to which SCG/GCG measurements have to be carried out through contact of a sensor with the subject's body.

The accessory is provided with at least one motion displacement sensor, such as an accelerometer, or a gyroscope, for measuring said kinematic characteristic or position of the subject's head.

As a consequence, the method according to the invention uses a space-reduced, non-invasive hardware.

The accessory is chosen preferably from the following: an eyewear frame, an eyewear addon, an eyewear clip, an eyewear holder strap cord, headphones, an earphone, a jewel.

The processing is advantageously performed in an embedded manner or on a remote host.

The invention further proposes a system for determining cardiac or respiratory activity of a subject, as claimed in claim 4.

Other advantageous and non-limiting features of the system for determining cardiac or respiratory activity of a subject according to the invention are:

- the accessory is chosen from the following: an eyewear frame, an eyewear addon, an eyewear clip, an eyewear holder strap cord, headphones, an earphone, a jewel;
- the processing unit is further programmed to implement, prior to recording the at least one measuring signal, the following steps:

  - determining whether sample measurements from the measurement unit satisfy a triggering condition,
  - in case the triggering condition is satisfied, increasing a sampling rate, a sampling duration and sensitivity of the measurement unit,
  - triggering the recording of the at least one measuring signal,

  wherein recording the at least one measuring signal is performed over a time slot included in the at least one period of time to obtain the sample measurements forming at least one recorded signal;
- when peaks are found during the quality evaluating step, the pre-processing phase further comprises a step consisting of reducing noise in the at least one selected windowed signal, to obtain at least one cleaned signal;
- step b) comprises after the pre-processing phase:

  - applying a first bandpass filter, a Hilbert transform, an envelope analysis and either a chirp z-transform or a numerical Fourier transform to the at least one cleaned signal to obtain at least one first power spectral density function,
  - identifying first spectral peaks in the at least one first power spectral density function,
  - evaluating the subject heart rate from the first spectral peaks;

- step b) comprises after the pre-processing phase:

  - applying a second bandpass filter and either a chirp z-transform or a digital Fourier transform to the at least one cleaned signal to obtain at least one second power spectral density function,
  - identifying second spectral peaks in the at least one second power spectral density function,
  - evaluating the subject respiratory rate from the second spectral peaks;

- when the measurement unit comprises at least one accelerometer and at least one gyroscope, the at least one first spectral density function comprises a first accelerometer power spectral density function originating from a measuring signal delivered by the at least one accelerometer and a first gyroscope power spectral density function originating from measuring signal delivered by the at least one gyroscope;
- identifying first spectral peaks comprises identifying accelerometer spectral peaks in the first accelerometer power spectral density function and gyroscope spectral peaks in the first gyroscope power spectral density function, and evaluating the subject heart rate comprises comparing a first relative band power of one chosen accelerometer spectral peak with a second relative band power of one chosen gyroscope peak;
- when the measurement unit comprises at least one accelerometer and at least one gyroscope, the at least one accelerometer being configured to measure temporal acceleration signals along three orthogonal axes, and said at least one gyroscope being configured to measure temporal angular rotational speed signals along three orthogonal axes, step b) comprises, after the pre-processing phase, implementing a principal component analysis on the temporal acceleration signals and on the temporal angular rotational speed signals, in order to evaluating said subject heart rate and said subject respiratory rate;
- step b) further comprises:

  - processing the at least one first power spectral density signal to obtain at least one seismocardiographic signal,
  - processing the at least one seismocardiographic signal to obtain at least one windowed aortic valve opening peak signal,
  - extracting local minima and maxima of the at least one windowed aortic valve opening peak signal,
  - annotating said local minima and maxima with fiducial points;

- step b) further comprises:

  - identifying respiratory cycles using the subject respiratory rate,
  - processing the respiratory cycles to obtain an inhalation volume of said subject, an estimation of a lung volume of said subject, an estimation of a lung capacity of the subject, an estimation of an inhalation phase and an exhalation phase of the subject.

DETAILED DESCRIPTION OF EXAMPLES

The following description with reference to the accompanying drawings will make it clear what the invention consists of and how it can be achieved. The invention is not limited to the embodiment/s illustrated in the drawings. Accordingly, it should be understood that where features mentioned in the claims are followed by reference signs, such signs are included solely for the purpose of enhancing the intelligibility of the claims and are in no way limiting on the scope of the claims.

In the accompanying drawings:

- figure 1 represents a comparison between a seismocardiographic (SCG) measurement and an electrocardiogram;
- figure 2 schematically a system for determining cardiac or respiratory activity of a subject according to the invention;
- figure 3 represents an eyewear frame according to an embodiment of the invention;
- figure 4 represents a measurement signal according to an embodiment of the invention;
- figure 5 schematically represents the main steps of the method for determining cardiac or respiratory activity of a subject according to the invention;
- figure 6 represents a pre-processed signal resulting from a processing step, according to the invention, of the measurement signal of figure 4;
- figure 7 represents an envelope signal of the pre-processed signal of figure 5 resulting from a processing step according to the invention;
- figure 8 represents the power spectral density of the envelope signal of figure 6 resulting from a processing step according to the invention;
- figure 9 represents an example of a power spectral density curve resulting from a processing step according to the invention;
- figure 10 represents a scatter plot of measurements taken with a system for determining the cardiac activity of a subject according to the invention;
- figure 11 represents measurement signals (raw and pre-processed) taken with a system according to the invention aiming at determining the respiratory activity of a subject;
- figure 12 represents the power spectral density of the pre-processed measurement signal of figure 11 resulting from a processing step according to the invention;
- figure 13 represents a flowchart describing a step of the method of characterizing the cardiac activity of a subject according to the invention;
- figure 14 represents a typical seismocardiographic waveform with fiducial points;
- figure 15 represents a detector signal according to the invention;
- figure 16 represents a signal presenting peaks detected by a thresholding method applied to the detector signal of figure 15;
- figure 17 represents the signal of figure 16 complemented by additional peaks retrieved by a processing step according to the invention;
- figure 18 represents the retrieval of aortic valve opening peaks resulting from the analysis of the signal of figure 17;
- figure 19 represents a seismocardiographic waveform obtained by processing steps according to the invention applied to the signal of figure 18;
- figure 20 represents an annotated version of the seismocardiographic waveform of figure 19;
- figure 21 represents a flowchart describing a step of the method of characterizing the respiratory activity of a subject according to the invention;
- figure 22 is a representation of human lung volumes and lung capacities.

Figure 2 illustrates schematically a system 1 for determining cardiac or respiratory activity of a subject according to the invention. The system 1 comprises an accessory 2 configured to be worn by the subject's head. The accessory 2 may be for instance an eyewear frame, headphones, an earphone, or a jewel. The accessory 2 is provided with a measurement unit 3 comprising at least one accelerometer 31 or at least one gyroscope 32. The measurement unit 3 may also be an inertial measurement unit comprising three accelerometers measuring linear acceleration values along three orthogonal axes, and three gyroscopes measuring rotational speeds around three orthogonal axes.

The at least one accelerometer 31 is for example a single-axis accelerometer, a dual-axis accelerometer, or a three-axis accelerometer. The at least one gyroscope 32 is for example a single-axis gyroscope, a dual-axis gyroscope, or a three-axis gyroscope.

The measurement unit 3 delivers at least one measuring signal reporting a kinematic characteristic or a position of the accessory 2 and, as a consequence, of the subject's head. The kinematic characteristic may be the speed along one direction, or a rotational speed around one direction, or an acceleration along one direction.

The accessory 2 also comprises a processing unit 4. The processing unit 4 is programmed to implement the following steps:

- receiving and recording the at least one measuring signal from the measurement unit 3 for obtaining sample measurements over at least one period of time,
- processing the sample measurements, forming at least one recorded signal, for determining said cardiac or respiratory activity.

The step of processing the sample measurements might be implemented in an embedded manner, that is to say, autonomously in the accessory 2. As a variant, the processing steps might be implemented remotely on a remote server receiving the sample measurements.

Figure 3 gives an illustration of an accessory 2 consisting of an eyewear frame, including the measurement unit 3 (not represented). The measurement unit 3 may beis located anywhere on the eyewear frame, such as in one of the temples,in the front face of the eyewear frame, or in one of the end tips of the eyewear frame. In this particular case, when the eyewear is a pair of connected glasses, the measurement unit 3 may advantageously be a sensor already embedded in the connected glasses, such as an inertial sensor used for physical activity measurement, typically the accelerometer of a pedometer. On figure 3, three orthogonal axes x, y and z are represented. The measurement unit 3 may then be a complete inertial measurement unit measuring linear acceleration values along those three axes and rotational speeds around those three axes.

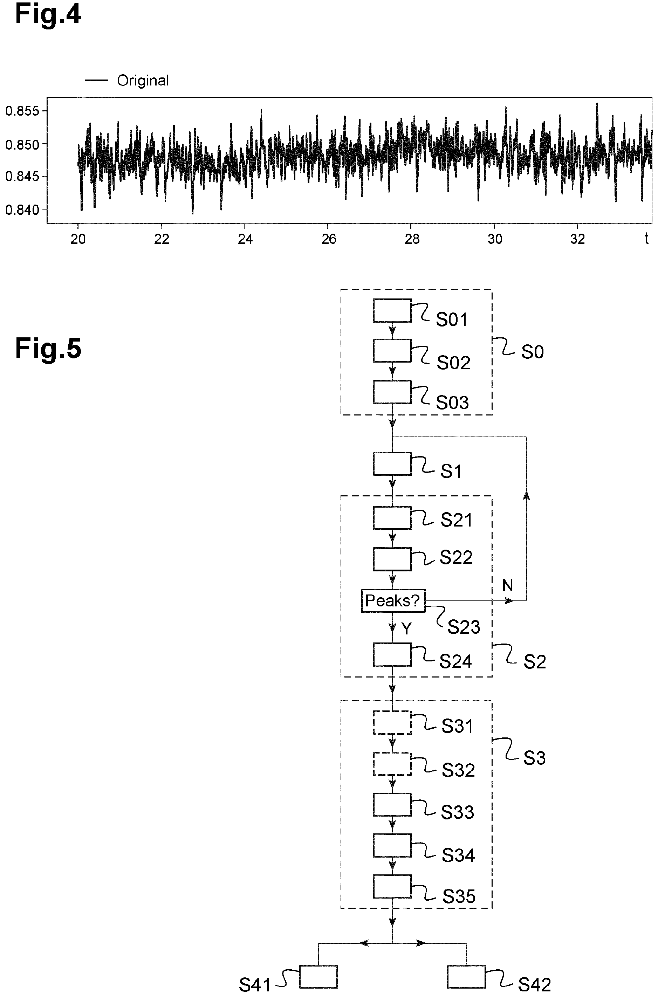

Figure 4 is an illustration of a typical raw signal (here an accelerometer signal along the z-axis of figure 3) obtained with the measurement unit 3.

Hence, the system 1 according to the invention allows, at will be shown in the following, obtaining measurements such as seismocardiographic measurements or gyrocardiographic measurements only by wearing the accessory on the head, in a permanently or semi-permanently manner, without having to position any sensor close to the heart and without, for the subject, having to be in a given position.

The processing of the sample measurements obtained from the measurement unit 3 of the system 1 aims at implementing a method for determining cardiac or respiratory activity of the subject wearing the accessory 2 on his head. By cardiac activity, it is meant both the heart rate and quantitative and qualitative description of the heartbeats of the subject. By respiratory activity, it is meant the respiratory rate and quantities such as the subject's inhalation volume and lung volume.

Figure 5 represents the main steps of the method for determining cardiac or respiratory activity of a subject wearing the accessory 2 of the system 1 on his head.

The determining method starts by a triggering step S0 aiming at triggering the acquisition of data by the measurement unit 3.

During this triggering step S0, it is first determined in a sub-step S01 whether the accessory 2 is worn by the subject. This can be performed either manually (the subject knows that he wears the accessory), or by detecting a level of activity of the subject. For instance, if the signal measured by the measurement unit 3 is sufficiently stable or repetitive, that is, exempt from noticeable activity, it can be inferred that the subject is for instance at rest, or walking, and thus, that measurements can be taken.

In case the accessory 2 is an eyewear frame, the indication that the signal measured by the measurement unit 3 is monotonic may be supplemented by a measurement of the eyewear frame orientation indicating that the eyewear frame is worn by the subject. For instance, using the axes of figure 3, the observation that the acceleration along the z-axis table might constitute this indication.

Then, in a sub-step S02, the sampling rate and the sensitivity of the measurement unit 3 are increased. In the case where the measurement unit 3 is an inertial measurement unit, the accelerometer ranges are set for example to 2g with a resolution of 0.122 mg and the gyroscope ranges are set to 2000 degrees per second with a resolution of 16.4 degrees per second. More accurate and sensitive inertial measurement units may be used as the measurement unit 3. A firmware embedded in the inertial measurement unit increases the sampling frequency from 5 Hz to for instance 200 Hz, or for instance to a sampling frequency between 100 Hz and 200 Hz.

Then in a sub-step S03, the acquisition of data by the measurement unit 3 is triggered.

As a variant, the triggering step S0 can be performed manually. For instance, the acquisition of data by the measurement unit 3 can be triggered on demand by the subject, either by activating a switch button, or with a remote request sent by the subject's mobile phone, or any other wearable device of the subject, so as to switch on the measurement unit 3.

The triggering step S0 is followed by an acquisition step S1, during which at least one measuring signal is recorded over a time slot. To determine the cardiac activity of the subject, the measuring signal is recorded for instance over a time slot of at least 10 seconds. To determine the respiratory activity of the subject, the measuring signal is recorded for instance over a time slot of at least 60 seconds.

The acquisition step S1 is followed by a pre-processing step S2, aiming at assessing the quality of the recorded signals for efficient extraction of the features of interest, that is, pertaining to the cardiac and respiratory activity of the subject.

The values constituting the recorded signals possess a timestamp, in units for instance of ticks, or milliseconds. In a sub-step S21 of the pre-processing step S2, the timestamp units are converted into seconds. After the time conversion of sub-step S21, the recorded signals are downsampled in a sub-step S22 by interpolation, for instance by linear, cubic, or polynomial interpolation. By way of example, to balance processing time and accuracy, a downsampling frequency of 100 Hz can be used. This value is suitable for analysing cardiac and breath activities, which significant signal features present a frequency lower than 50 Hz.

After the downsampling sub-step S22, a sub-step S23 is performed, consisting of a search for peaks in the recorded signals. The search for peaks is performed using a thresholding algorithm.

For instance, the search for peaks detects peaks of prominence 0.2. The prominence of a peak measures how much the peak stands out due to its intrinsic height and its location relative to other peaks. To measure the prominence of a peak P, the procedure described below can be followed: placing a marker on the peak P; extending a horizontal line from the peak P to the left and right until the line either crosses the signal because there is a higher peak, or reaches the left or right end of the signal; finding the minimum of the signal in each of the two intervals defined (left and right), this minimum of the signal being either a valley or one of the signal endpoints; the higher of the two interval minima specifies the reference level $L_{ref}$. The height of the peak P above this reference level $L_{ref}$ is its prominence.

If no peak is found in sub-step S23, that is to say that the recorded signal only comprises noise, the method goes back to the acquisition step S1 in order to acquire signals of better quality.

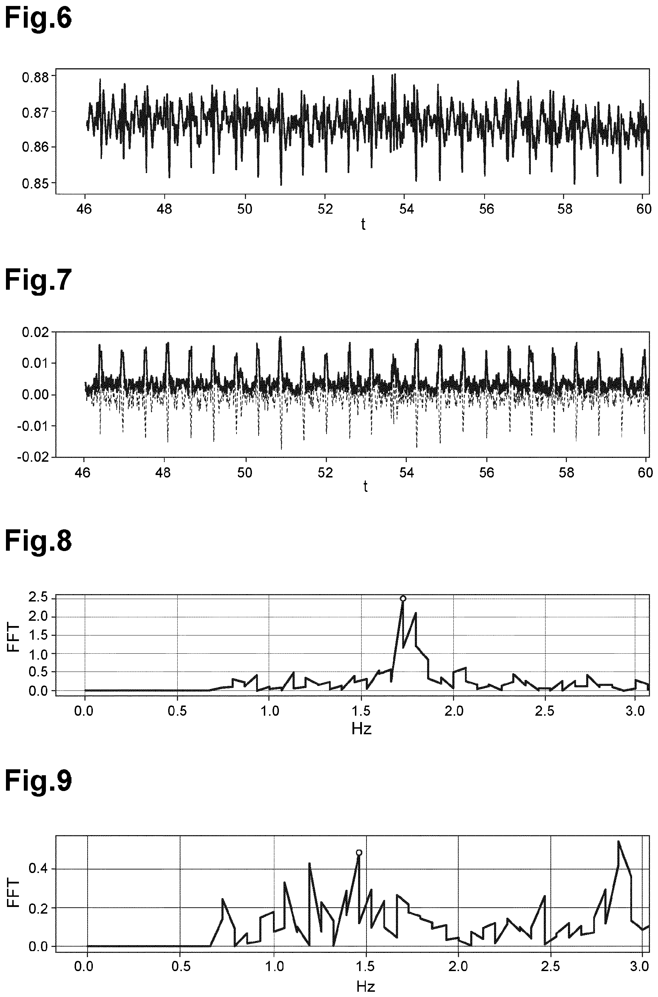

If peaks are found during the sub-step S23, a sub-step S24 is performed consisting of reducing noise in the recorded signals. The noise reduction of sub-step S24 is performed on so-called "best time windows". Those best time windows are portions of the recorded signals with a predetermined time duration selected in the time slot over which the recorded signals are recorded. The best time windows are time windows where the majority of the peaks have globally the same amplitude and where artefacts in the recorded signals are minimized. Figure 6 shows an example of a pre-processed signal in a chosen best time window. The signal is a temporal signal $a_z(t)$, t denoting the time, measured by an accelerometer 31 of the measurement unit 3.

Then, once a best time window is chosen, noise is reduced on the corresponding portion of the recorded signal, noted $S_{BEST}(t)$, by applying to it a filter, for instance a moving average filter of order 3 or 5, or a band-pass filter, or a combination of a low pass and a high-pass butterworth filter with, for instance, respective cutting frequencies at 0.1 Hz and 50 Hz.

The noise reducing filter parameters vary when considering either the cardiac activity estimation or the respiratory activity estimation.

For instance, a band-pass filter with cutting frequencies of 4Hz and 50 Hz might be applied on the fast Fourier transform of the signal $S_{BEST}(t)$ for the cardiac activity estimation, while a band-pass filter with cutting frequencies of 0.1 Hz and 0.9 Hz might be applied for the respiratory activity estimation. Indeed, typically, the bandwidth of the applied filter for heart rate estimation is configured to focus on the heart rate range comprised between 40 beats per minute and 240 beats per minute, while the bandwidth of the applied filter for respiratory rate estimation is configured to focus on the respiratory rate range comprised between 6 breaths per minute and 40 breaths per minute.

Artefacts due to involuntary or voluntary movements of the subject's body, such as walking, speaking, yawning, may be additionally removed by using moving average filters, polynomial smoothing, Savitzky-Golay filtering, median filters, comb filtering, empirical mode decomposition.

The pre-processing step S2 ends when the noise reduction sub-step S24 is performed.

Then a rate estimation step S3 (heart rate $R_H$ and/or respiratory rate $R_R$) is carried out following the sub-steps described below.

It is supposed here that one signal, either an accelerometer signal $a_{BEST}(t)$, or a gyroscope signal $g_{BEST}(t)$ is available (t being the time in seconds), corresponding to the signal obtained after the pre-processing step S2 has been implemented.

In the case of the subject's respiratory rate $R_R$ estimation, the following sub-steps S31 and S32 are optional.

In a sub-step S31, the Hilbert transform of the signal $a_{BEST}(t)$ (respectively $g_{BEST}(t)$) is computed. The Hilbert transform of a temporal function h(t) is defined by:

$$\tilde{h}(t) = -\int_0^\infty [b(f)\ sin\ (ft) - c(f)\ cos\ (ft)\ ]df,$$

with

$$b(f) = \frac{1}{\pi}\int_{-\infty}^\infty h(t)\ cos\ (ft)dt \ \text{ and } c(f) = \frac{1}{\pi}\int_{-\infty}^\infty h(t)\ sin\ (ft)\ dt.$$

6

The output of sub-step S31 is a signal $\widetilde{a_{BEST}}(t)$ (respectively $\widetilde{g_{BEST}}(t)$ ).

In a sub-step S32, an envelope analysis of the Hilbert transform $\widetilde{a_{BEST}}(t)$ , respectively $\widetilde{g_{BEST}}(t)$ is then computed, outputting a signal $A_{BEST}(t)$, respectively $G_{BEST}(t)$. Figure 7 illustrates in plain line the signal $A_{BEST}(t)$ obtained after implementation of sub-step S31 and S32 to the signal $a_{BEST}(t)$ of figure 6 (in dotted line).

In a sub-step S33, a chirp z-transform or a discrete Fourier transform (DFT), for instance implemented by a fast Fourier transform (FFT) computation, is applied to the signal $A_{BEST}(t)$, respectively $G_{BEST}(t)$, (or if sub-steps S31 and S32 were not performed, to the signal $a_{BEST}(t)$, or $g_{BEST}(t)$), outputting a signal F(A) (f), respectively F(G) (f), f denoting the frequency in Hz.

The expression of the chirp z-transform of a sampled signal h(n), n=0..N-1 is given below:

$$H_k = \sum_{n=0}^{N-1} h(n)z_k^{-n} \text{ for k=0,1..M-1}$$

and where $z_k = A * W^{-k}$ for k=0,1..M-1, A denoting a complex starting point, W being the complex ratio between points $z_j$, and M being the number of points.

The expression of the digital Fourier transform of a sampled signal h(n), n=0,1.. N-1 comprising N samples is given below:

$$H_k = \sum_{n=0}^{N-1} h(n)e^{-i2\pi k\frac{n}{N}} \text{ for k=0,1..N}$$

In a sub-step S34, the power spectral density DSPA(f), respectively, DSPG(f), is computed from the signal F(A) (f), respectively F(G)(f):

$$DSPA(f) = \frac{1}{T}|F(A)(f)|^2$$

$$DSPG(f) = \frac{1}{T}|F(G)(f)|^2$$

where T is the averaging time interval.

Other ways to compute the power spectral density DSPA(f), respectively, DSPG(f), may be by computing the autocorrelation of the signal F(A)(f), respectively F(G)(f) or by using the Welch's method.

Figure 8 gives an example of a power spectral density curve obtained after implementation of a sub-step S34, where the frequency corresponding to the maximal power spectral density is indicated with a black circle (approximately 1.7 Hz).

In a sub-step S35, the heart rate $R_H$, respectively, the respiratory rate $R_R$, are estimated by first identifying the frequency $f_{max}$ with the maximal power spectral density in the signal DSPA(f), respectively, DSPG(f), then discriminating a fundamental frequency associated with higher order harmonics in the following manner.

Indeed, sometimes, the first harmonic of a signal can have a higher power spectral density than the fundamental frequency. The solution used in the present invention consists in considering two portions P1 and P2 of the power spectral density curve centered around respectively the frequencies $f_{max}/2$ and $f_{max}$ and presenting each one a bandwidth of width $f_{max}/2$. Then, the ratio between the area below the power spectral density curve in the portion P1 and the area below the power spectral density curve in the portion P2 is computed. As implemented in the present disclosure, the ratio is compared to the value 0.01. If the ratio is smaller than 0.01, it is inferred that the rate to be estimated, heart rate $R_H$, respectively, respiratory rate $R_R$, corresponds to the frequency $f_{max}$. If the ratio is higher than 0.01, a search for a peak of the power spectral density curve around the frequency $f_{max}/2$ is performed. If a peak is found close to the frequency $f_{max}/2$, it is inferred that the rate to be estimated corresponds to the frequency $f_{max}/2$.

Figure 9 illustrates this discrimination method: it can be seen that the power spectral density curve presents a maximal peak at a frequency of about $f_{max}$ equal to 2.86 Hz. Then, following the method above, two frequency ranges are considered: a bandwidth of 1.43 Hz centered respectively at $f_{max}/2$ and at $f_{max}$. The ratio of the areas is found greater than 0.01 and a peak centered at 1.46 Hz is detected in the first frequency range, illustrated by a black circle, very close to the frequency $f_{max}/2$. Thus, the final estimation is evaluated to 1.46 Hz. To evaluate the searched rate, the following formulas are used:

$$R_H \text{ (bps)} = f_{max} \text{ (Hz) and } R_H \text{ (bpm)} = 60 \, f_{max,}$$

with bps the abbreviation for beats per seconds and bpm the abbreviation for beats per minute.

In the previous example, the frequency of value 1.46 Hz corresponds to a heartbeat rate of 88 bpm.

The rate estimation step S3 ends after the sub-step S35 is performed.

Table 1 below shows a series of results obtained in the estimation on several samples of signals. The results compare reference values of heart rates and values of heart rates obtained with the method according the invention with the system 1.

Table 1

| Sample | HR Reference (bpm) | HR measurement (bpm) | HR Reference - HR measurement (bpm) |
|---|---|---|---|
| 1 | 80 | 80 | 0 |
| 2 | 76 | 72 | 4 |
| 3 | 75 | 72 | 3 |
| 4 | 88 | 76 | 12 |
| 5 | 69 | 68 | 1 |
| 6 | 78 | 72 | 6 |
| 7 | 130 | 124 | 6 |
| 8 | 130 | 128 | 2 |
| 9 | 110 | 108 | 2 |
| 10 | 106 | 104 | 2 |
| 11 | 65 | 64 | 1 |
| 12 | 102 | 108 | -6 |
| 13 | 90 | 88 | 2 |

The resulting average error is 2.69 bpm and the standard deviation is 4.09 bpm.

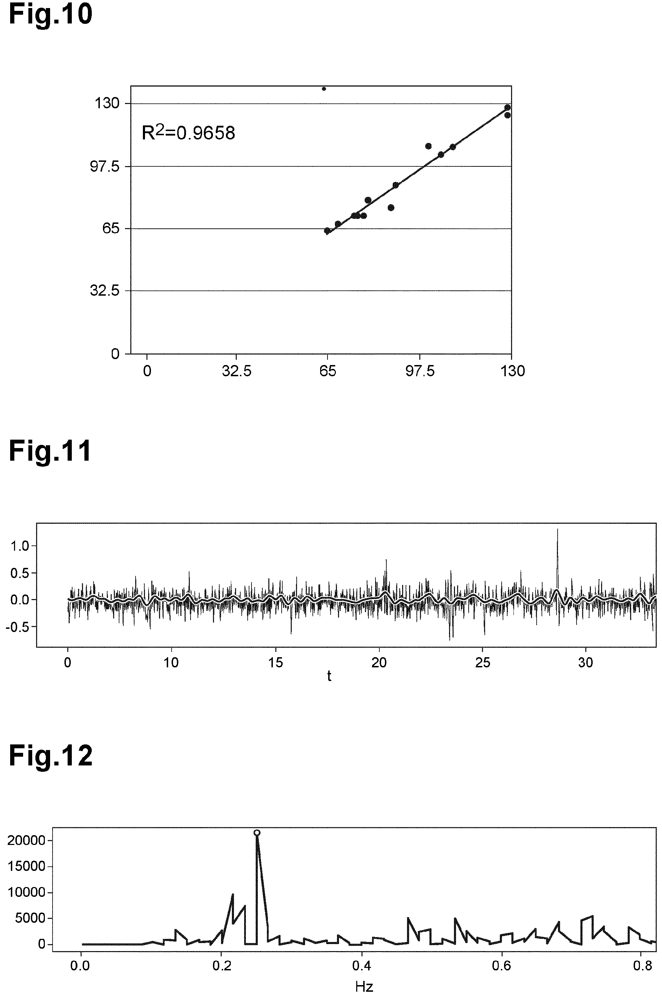

Figure 10 shows the scatter plot corresponding to those measurements, fitted with a linear regression presenting an $R^2$ correlation coefficient of 0.97.

In a preferred embodiment, when the measuring unit 3 comprises an accelerometer measuring at least a signal $a_z$(t) along the z-axis of figure 3, the signal $a_z$(t) is used as the recorded signal processed in steps S0, S1, S2 and S3.

Figures 11 and 12 give an illustration of signals used for the estimation of the respiratory rate $R_R$ of the subject.

In a preferred embodiment, for estimating the heart rate $R_H$ of the subject, a signal coming from an accelerometer 31 of the measuring unit 3, specifically measuring acceleration values along the z-axis of figure 3 is used for the implementation of step S0 to S3.

Figure 11 shows an example of temporal signal used for the determination of the respiratory rate estimation and its noise-reduced version (smooth black curve with a white border) after application of sub-step S24. Here, more precisely, a band-pass filter with cutting frequencies of 0.1 Hz and 0.9 Hz has been applied to the blue curve of figure 11.

Figure 12 shows the corresponding power spectral density curve obtained after implementation of the power spectral density computation sub-step S34. On this figure, the frequency corresponding to the maximal power spectral density may be evaluated, here at 0.25 Hz, as indicated by the black circle. The corresponding respiratory rate is then 15 breaths per minute.

In an embodiment, in the case of the heart rate $R_H$ estimation, when both an accelerometer signal and a gyroscope signal are available, those two signals might be combined to increase the rate estimation accuracy.

The combination is performed according to the following decision algorithm.

If the frequency $f_a$ resulting from the accelerometer signal analysis and the frequency $f_g$ resulting from the gyroscope signal analysis are close values, their average is chosen as the searched rate:

$$R_H = \frac{f_a + f_g}{2}$$

If the peak at the frequency $f_a$ resulting from the accelerometer signal analysis presents a relative power band power higher than 0.9, and the peak at the frequency $f_g$ resulting from the gyroscope signal analysis presents a relative band power smaller than 0.9, the heart rate $R_H$ in beat per second corresponding to the frequency $f_a$ is chosen as the searched rate. More specifically, with the frequency $f_a$ given in Hz:

$$R_H \text{ (bps)}=f_a \text{ and } R_H \text{ (bpm)}=60 \ f_a$$

By "relative band power" of the peak at the frequency $f_a$ or of the peak at the frequency $f_g$, it is meant the power in a frequency band corresponding to the peak at the frequency $f_a$ computed as a percentage of the total power of the signal, respectively, in a frequency band corresponding to the peak at the frequency $f_g$. The frequency band is computed from the width of the peak at the frequency $f_a$, respectively, $f_g$.

If the peak at the frequency $f_a$ resulting from the accelerometer signal analysis presents a relative band power smaller than 0.9, and the peak at the frequency $f_g$ resulting from the gyroscope signal analysis presents a relative band power higher than 0.9, the heart rate $R_H$ in beats per second corresponding to the frequency $f_g$ is chosen as the searched rate.

$$R_H \text{ (bps)}=f_g \text{ and } R_H \text{ (bpm)}=60 \ f_g$$

Otherwise, if a previous estimation of the heartbeat rate was shortly performed, the frequency among $f_a$ and $f_g$ being the closest to the previously estimated heart rate $R_H$ is chosen.

Otherwise, if the peak at the frequency $f_g$ resulting from the gyroscope signal analysis presents a relative band power higher than the relative band power of the peak at the frequency $f_a$ resulting from the accelerometer signal analysis, for instance by a factor of 3, it means that the estimation from the gyroscope is 3 times clearer than that from the accelerometer. Therefore, the rate corresponding to the frequency $f_q$ is chosen as the searched rate.

Otherwise, the rate corresponding to the frequency $f_a$ resulting from the accelerometer signal analysis is chosen as the searched rate.

In an embodiment, , when three accelerometer signals, $a_x(t)$, $a_y(t)$ and $a_z(t)$, and three gyroscope signals $g_x(t)$, $g_y(t)$ and $g_z(t)$ are available (with x, y and z being related for instance to the axes represented in Figure 3), they might be combined to increase the heart rate $R_H$ accuracy or the respiratory rate $R_R$ estimation accuracy.

It is assumed that the signals $a_x(t)$, $a_y(t)$, $a_z(t)$ on one hand, and $g_x(t)$, $g_y(t)$, $g_z(t)$ on the other hand, are the signals obtained after the sub-step S23 and that peaks were found in those signals.

Their combination is performed by using a principal component analysis (PCA) to determine the best direction to capture the most variance.

The PCA principle consists in fitting a p-dimensional ellipsoid to data, here, the signals $a_x(t)$, $a_y(t)$, $a_z(t)$ on one hand, and $g_x(t)$, $g_y(t)$, $g_z(t)$ on the other hand. Each of the p axes of the ellipsoid is characterized by a variation value, that is, the total distance among the projected data, and by a variance value, that is, the total distance from the original data to their corresponding projected data on the axis. The axis with the most variance corresponds to the representation that is the closest to the original data.

The list of measured vectors (ax(t), ay(t), az(t)) and (gx(t), gy(t), gz(t)) is projected on the best direction axis found by the principal component analysis, in order to output a single signal for each triplet, respectively $a_{principal}(t)$ and $g_{principal}(t)$.

The noise-reducing sub-step S24 is then performed by applying to both signals $a_{principal}(t)$ and $g_{principal}(t)$ a moving average filter of order 5, followed by either an ideal band-pass filter or the combination of a low pass with a 0.1 Hz cutting frequency and a high pass filter with a 0.5 Hz cutting frequency. The sub-step S24 outputs two noise-reduced signals $S_a(t)$ (from the accelerometer measurements) and $S_g(t)$ (from the gyroscope measurements).

In this embodiment, the respiratory rate estimation step S3 is performed differently from the series of sub-steps S31 to S35 previously described, as follows. Four sub-steps S31b to S34b are applied to each of the signals $S_a(t)$ and $S_g(t)$.

In a sub-step S31b, the signal to noise ratio $SNR_a$, respectively $SNR_g$, of the noised-reduced signal $S_a(t)$, respectively $S_d(t)$, is computed.

In a sub-step S32b, the power spectral density of the signal $S_a$, respectively $S_g$ is computed using the chirped z-transform or a discrete Fourier transform, for instance implemented by a fast Fourier transform (FFT) computation, of those signals $F(S_a)$ (f), respectively $F(S_g)$ (f):

$$DSPA(f) = \frac{1}{T}|F(S_a)(f)|^2$$

$$DSPG(f) = \frac{1}{T}|F(S_g)(f)|^2.$$

In a sub-step S33b, peaks are identified and sorted in a descending order using the value of the power spectral density as a weight. Any peak searching method may be used, such as as correlation, filtering, demodulation, sliding average.

In a sub-step S34b, the greater weighted value is selected as the respiratory rate frequency. $f_{Ra}$, respectively $f_{Rg}$.

In a final sub-step S35, a discrimination is performed between the two results $f_{Ra}$ and $f_{Rg}$ by choosing the result $f_R$ corresponding to the highest signal to noise ratio computed at the sub-step S31b.

Similarly to the heart rate estimation, the respiratory rate is given by:

$$R_R \text{ (bps)} = f_R \text{ (Hz)}$$

$$R_R \text{ (bpm)} = 60*f_R \text{ (Hz)}$$

In a preferred embodiment, for estimating the respiratory rate $R_R$ of the subject, a signal coming from a gyroscope 32 of the measuring unit 3 is used for the implementation of steps S0 to S3.

Once the heart rate $R_H$ of the subject and/or the respiratory rate $R_R$ of the subject have been estimated at the end of step S3, specific features of interest can be extracted to characterize both the heart activity and the respiratory activity.

In what follows, two separate steps S41 and S42 for characterizing respectively the heart activity and the respiratory activity of the subject, after having estimated his heart rate $R_H$ and his respiratory rate $R_R$, are described.

First, a step S41 for characterizing the heart activity of the subject will be described.

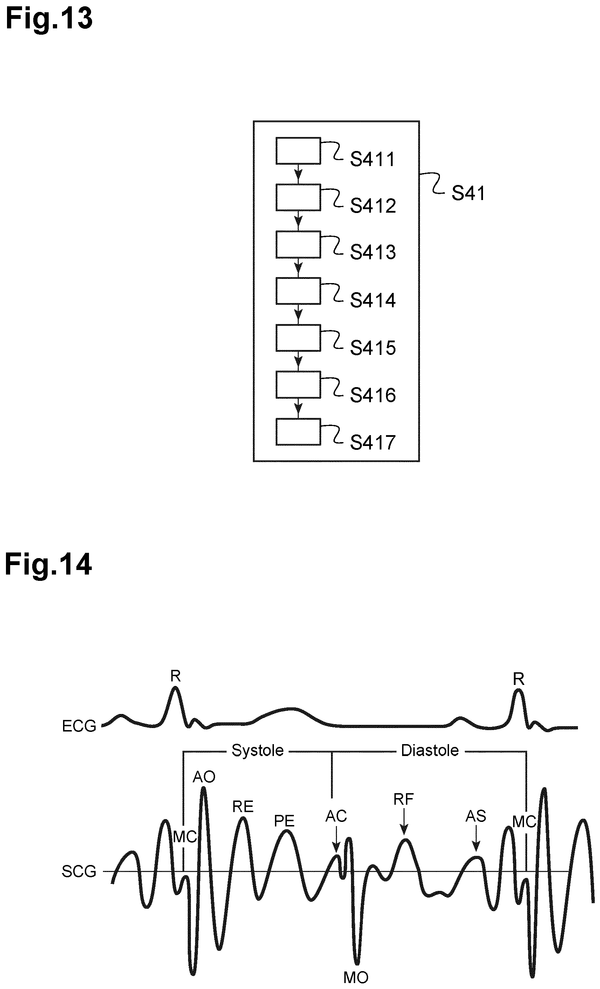

Figure 13 is a flowchart illustrating schematically step 41 and will be described in what follows.

A typical seismocardiogram is represented at figure 14 with points of interest between two heart beats. Both systolic points and diastolic points may be reported on this typical seismocardiogram. Those points of interest cannot be extracted from a traditional electrocardiogram. The systolic points comprise the aortic valve opening (AO), the peak ventricular ejection (PE), the aortic valve closure (AC). The diastolic points comprise the mitral valve opening (MO), the rapid ventricular filling (RF), the atrial systole (AS) and the mitral valve closure (MC).

The step S41 aims at indexing those points on a signal typically measured with the measuring unit 3.

It is supposed here that one temporal signal SCG(t), corresponding to one among an accelerometer signal $a_{BEST}$(t), or a gyroscope signal $g_{BEST}$(t) as previously defined, is available (t being the time in seconds), and obtained after step S2 has been implemented. For instance, the signal SCG(t) may have undergone a bandpass filter of cutting frequencies 4Hz and 50 Hz. It is also supposed that the heartbeat rate $R_H$ in beats per second (bps) has been estimated after implementation of step S3. The heartbeat rate $R_H$ corresponds to the rate between two aortic valve opening peaks (AO) in the SCG(t) signal.

In a first sub-step S411, a detector signal $x_{det}$(t) defined by the formula below is computed in order to detect AO peaks:

$$x_{det}(t) = \left| \frac{d^2 SCG(t)^3}{dt} \right|$$

Optionally, in the formula of the detector signal $x_{det}$(t), either only the negative part of the SCG(t), or only the positive part of the SCG(t) signal may be considered to compute the detector signal $x_{det}$(t).

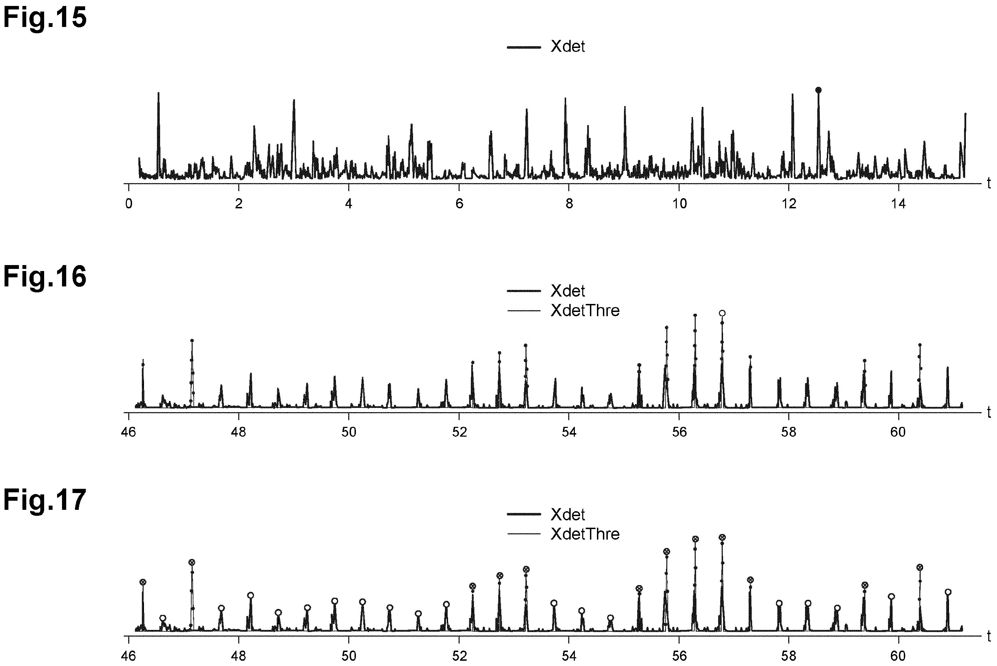

Figure 15 illustrates an example of the shape of the $x_{det}$(t) signal, with, indicated by the red dot, its maximum value.

Optionally, the signal $x_{det}$(t) may be smoothed by applying to it a moving average filter (for instance of order 5).

In a next sub-step S412, the $x_{det}$(t) signal is divided into segments, using the time index of its maximum value and the estimated heartbeat rate $R_H$ in bps. More specifically, the signal $x_{det}$(t) is divided into segments of duration Dt=1/$R_H$ and starting at times $t_{max}$- 0.215 Dt, where $t_{max}$ denotes the time index of the maximum value of the $x_{det}$(t) signal.

In a next sub-step S413, the local maximum of each segment is stored.

In a next sub-step S414, a thresholding operation at the 60th percentile is performed, outputting peaks which magnitude is higher than 60% of the magnitude of each local maximum. Figure 16 shows the superposition of the signal $x_{det}$(t) versions before and after thresholding. The peaks obtained after the thresholding operation are represented by the curve with dots.

In a next sub-step S415, intervals between all pairs of consecutive peaks in the thresholded signal $x_{det}$(t) are computed.

The following estimation algorithm for each computed interval I is then implemented.

In case I is lower than 1/$R_{Hmax}$, where $R_{Hmax}$ denotes the greatest heart rate value allowed (typically $R_{Hmax}$ is equal to 200 bpm): the peaks are too close, thus only the peak with the greatest value is considered. This case allows detecting outlier peaks.

In case I is greater than 1.2*Dt, it is inferred that peaks are missing in the corresponding interval. Retrieval of the missing peaks is performed under the assumption that they are regularly distributed in the interval I. Thus, missing peaks are estimated theoretically. To help the retrieval, measured peaks close to the theoretically estimated peaks are used.

Figure 17 illustrates the additional detected peaks (represented by black empty circles) in the signal $x_{det}(t)$ comprising already the peaks obtained after the thresholding operation of sub-step S414 (represented by black filled circles).

Otherwise, the interval is considered acceptable, and the two corresponding peaks, that is, defining the interval, are considered and kept for analysis.

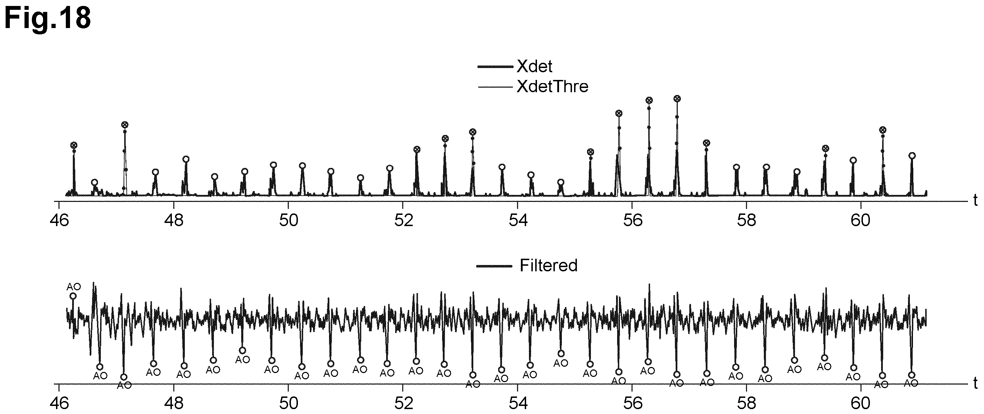

Figure 18 shows the correspondence between the peaks detected after the implementation of the estimation algorithm previously described, and the AO peaks. More specifically, an AO peak is associated with each detected peak in the signal $x_{det}(t)$.

It can be remarked that the AO peaks may be used to evaluate the Heart rate Variability (HRV) of the subject. The HRV is the physiological phenomenon of variation in the time interval between heartbeats. It is measured by the variation in the beat-to-beat- intervals. The HRV is a good indicator for cardiovascular and non-cardiovascular diseases.

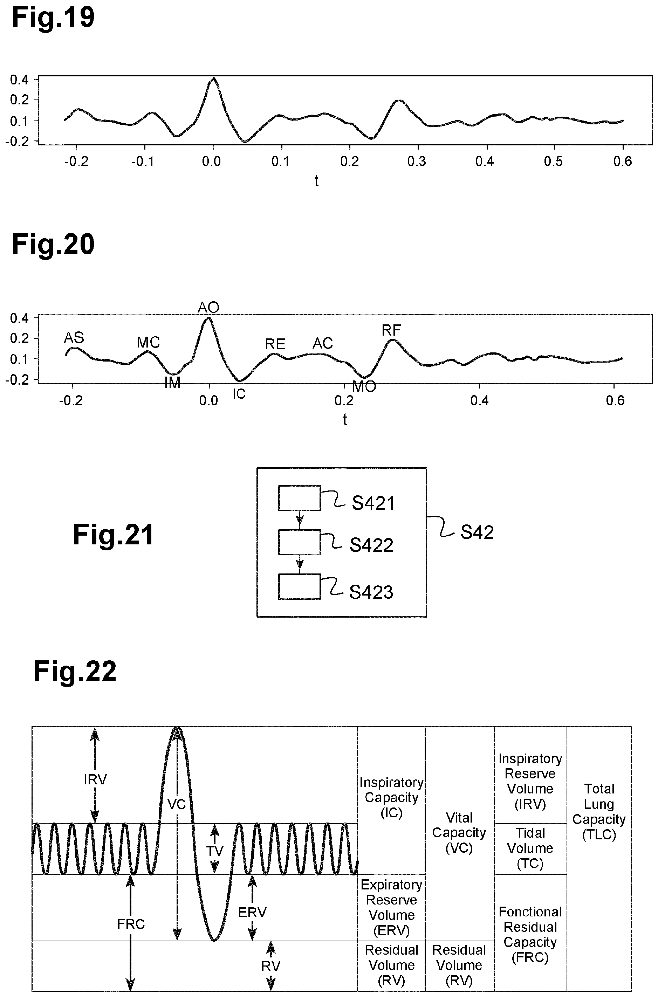

In a sub-step S416, the signal SCG(t) is segmented. The signal is normalized in magnitude in each segment in order to compute the median curve $SCG_{median}(t)$ among all segments. Figure 19 is an example of a median curve obtained as a result of sub-step S416. On this curve, the point of abscissa 0 corresponds to the median of all AO peaks.

In a sub-step S417, the fiducial points of a typical seismocardigram are identified on the median curve $SCG_{median}(t)$ in following manner. A window of duration Dt and starting at around $t_{AO}$-0.25 Dt is extracted from the median curve $SCG_{median}(t)$, $t_{AO}$ denoting the time index of the AO peak. Local minima and maxima are then extracted to annotate the signal. Figure 20 illustrates an annotated median curve.

Typically, the sequence IM-AO-IC-RE is used to discriminate cases where the amplitude of the diastole moment is greater than that of the systole moment.

As a variant, more than one signal $S_{BEST}(t)$ may be used, that is to say that several best windows might be taking into account in order to smooth the median curve and to increase the signal to noise ratio. For instance, for each new best window acquisition signal $S_{BEST\_i}(t)$, an associated heartbeat rate $R_{Hi}$ can be computed. Then, all signals may be temporally scaled in accordance to the $R_{Hi}$ values, so as to obtain a common reference.

As another variant, machine learning algorithms, using for instance neural networks, may be used to perform the fiducial points identification.

Now, a step S42 for characterizing the respiratory activity of a subject will be described.

Figure 21 is a flowchart illustrating schematically step 42 and will be described in what follows.

It is supposed here that one temporal signal $S_R(t)$, corresponding to one among an accelerometer signal $a_{BEST}(t)$, or a gyroscope signal $g_{BEST}(t)$ as previously defined, is available (t being the time in seconds), and obtained after the pre-processing step S2 has been implemented. For instance, the signal SCG(t) may have undergone a band-pass filter with cutting frequencies of 0.1 Hz and 0.9 Hz. It is also supposed that the respiratory rate $R_R$ in beats per second (bps) has been estimated.

Typical quantities of interest are the subject's inhalation volume and his lung capacity. Air in the lungs is measured in terms of lung volume and lung capacity. The volume measures the amount of air for one function such as inhalation or exhalation. The capacity is any two or more volumes. An example of capacity if how much air can be inhaled starting from the end of a maximal exhalation.

Figure 22 is an illustration of human lung volumes and capacities. The total lung capacity TLC is the volume of air contained in the lungs at the end of a maximal inspiration. The inspiratory capacity IC is the maximum volume of air that can be inspired after reaching the end of a normal, quiet expiration. The inspiratory reserve volume IRV is the extra volume of air that can be inspired with maximal effort after reaching the end of a normal, quiet inspiration. The tidal volume TV is the volume of air inspired or expired during each normal, quiet respiratory cycle. The IC is the sum of the IRV and the TV. The functional residual capacity FRC is the volume of air remaining in the lungs at the end of a normal, quiet expiration. The FRC is the sum of the ERV and the RV. The expiratory reserve volume ERV is the extra volume of air that can be expired with maximum effort beyond the level reached at the end of a normal, quiet expiration. The residual volume RV is the volume of air remaining in the lungs at the end of a maximal expiration. Figure 22 shows how those different volumes and capacities can be retrieved by analysing a temporal signal representing the respiratory over a period of time.

In a first sub-step S421, respiratory cycles are identified in the temporal signal $S_R(t)$. More specifically, sinusoids of temporal period $1/R_R$ are fitted in the temporal signal $S_R(t)$. The inhalation volume is estimated by combining the amplitude of the fitted sinusoids with an estimation of the subject's lung volume based on his height.

In a next sub-step S422, the subject's lung capacity is estimated in the following manner. First, the Hilbert transform of one identified respiratory cycle is computed. The subject's lung capacity is estimated as the mean value of the analytic envelope of the Hilbert transform previously computed. In a variant, to increase the estimation accuracy, a weighted average of different values obtained for different respiratory cycles may be used.

In a next sub-step S423, respiratory phases, that is, the inhalation phase and the exhalation phase might be identified.

The inhalation phase may be predicted thanks to the combination of an instantaneous phase evaluation in the Hilbert transform of the lowest frequency components of the temporal signal $S_R(t)$. The instantaneous phase is then correlated to the phase of the peak frequency of the respiratory power spectral density DSPG(f).

The phase of the sinusoids fitted at sub-step S421 gives information about the respiratory state of the subject, that is, inhaling or exhaling, whatever the point on the respiratory cycle.

Therefore, the system 1 according to the invention allows estimating the cardiac or the respiratory activity of the subject wearing the accessory 2, in a permanent or semi-permanent manner, without any constraint of position of the accessory or of the subject itself. The solution proposed by the invention is cost-effective, low-power consuming and space-reduced.

Advantageously, while for instance classical SCG techniques require the sensor being close to the chest to obtain a clear signal, the system 1 according to the invention is able to extract an estimate of the cardiac or the respiratory activity of a subject, on the basis of measurements performed far from the heart and the chest.

As the system 1, through the accessory 2, is worn permanently or semi-permanently, suitable time slots to perform measurements acquisitions may be selected.

Finally, the measuring unit 3 providing the signals for cardiac and/or respiratory activity estimation is not restricted to this application but might be shared for other functionalities of the accessory 2, such as step counting, posture monitoring, power management,...

Variants:

The processing performed in steps S2, S3, S41 and S42 may be applied to any signal or any combination of signals coming from the accelerometer 31 and/or the gyroscope 32 of the measuring unit 3. For instance, steps S2, S3, S41 and S42 might be applied to the signal with minimum noise, or, when an inertial measurement unit comprising three accelerometers and three gyroscopes is used as the measurement unit 3, to the magnitude of the vector $(a_x, a_y, a_z)$ or $(g_x, g_y, g_z)$, to a weighted average of the signals $a_y$, $a_y$, and $a_z$, and $g_y$, $g_y$, and $g_z$, or to any linear or non-linear combinations of those signals.

Other signal processing methods such as direction peaks detection, polynomial spline fit, wavelet transform, empirical mode decomposition may be used to remove noise and artefacts linked to the subject's body motion, or to replace the computation of the Fourier transform in the estimation of the heart rate $R_H$ or of the respiratory rate $R_R$.

**Claims**

1. Method for determining cardiac or respiratory activity of a subject, comprising a step of measuring and recording, over at least one period of time, sample measurements relating at least to a kinematic characteristic or a position of an accessory (2) worn by the subject's head and a step of processing by a processing unit those sample measurements for determining said cardiac or respiratory activity

   **characterized in that** the step of processing comprises a pre-processing phase with the following steps:

   - converting timestamps of said sample measurements into chosen temporal units,
   - downsampling a sampling frequency of said at least one recorded signal to obtain at least one downsampled signal,
   - applying at least one window function to the at least one downsampled signal to obtain at least one windowed signal,
   - choosing at least one selected windowed signal among the at least one windowed signal,
   - estimating quality of said at least one selected windowed signal by searching for peaks in said at least one selected windowed signal.

2. Method according to claim 1, wherein said accessory (2) is provided with at least one motion sensor, such as an accelerometer (31) or a gyroscope (32) for measuring said kinematic characteristic or position of the subject's head.

3. Method according to one of claims 1 to 2, wherein said processing is performed in an embedded manner or on a remote host.

4. System (1) for determining cardiac or respiratory activity of a subject, said system (1) comprising an accessory (2) which is configured to be worn by the subject's head and which is provided with a measurement unit (3) comprising at least one motion sensor, such as an accelerometer (31) or a gyroscope (32) and delivering at least one measuring signal reporting at least a position or a speed or a rotational speed or an acceleration of the accessory (2) and a processing unit (4) programmed to implement the steps of:

- a) receiving and recording said at least one measuring signal from the measurement unit (3) for obtaining sample measurements over at least one period of time,
- b) processing said sample measurements forming at least one recorded signal for determining said cardiac or respiratory activity

**characterized in that** step b) comprises a pre-processing phase with the following steps:

- converting timestamps of said sample measurements into chosen temporal units,
- downsampling a sampling frequency of said at least one recorded signal to obtain at least one downsampled signal,
- applying at least one window function to the at least one downsampled signal to obtain at least one windowed signal,
- choosing at least one selected windowed signal among the at least one windowed signal,
- estimating quality of said at least one selected windowed signal by searching for peaks in said at least one selected windowed signal.

5. System (1) according to claim 4, wherein the accessory (2) is chosen from the following: an eyewear frame, an eyewear add-on, an eyewear clip, an eyewear holder strap cord, headphones, an earphone, a jewel.

6. System (1) according to claim 4 or claim 5, wherein the processing unit (4) is further programmed to implement, prior to recording said at least one measuring signal, the following steps:

- determining whether sample measurements from the measurement unit (3) satisfy a triggering condition,
- in case the triggering condition is satisfied, increasing a sampling rate, a sampling duration and sensitivity of the measurement unit (3),
- triggering the recording of said at least one measuring signal,

and wherein recording said at least one measuring signal is performed over a time slot included in the at least one period of time to obtain said sample measurements forming at least one recorded signal.

7. System (1) according to any one of claims 4 to 6, wherein when peaks are found during the quality evaluating step, said pre-processing phase further comprises a step consisting of reducing noise in the at least one selected windowed signal, to obtain at least one cleaned signal.

8. System (1) according to claim 7, wherein step b) comprises after the pre-processing phase:

- applying a first bandpass filter, a Hilbert transform, an envelope analysis and either a chirp z-transform or a numerical Fourier transform to the at least one cleaned signal to obtain at least one first power spectral density function,
- identifying first spectral peaks in the at least one first power spectral density function,
- evaluating said subject heart rate ($R_H$) from said first spectral peaks.

9. System (1) according to claim 7, wherein step b) comprises after the pre-processing phase:

- applying a second bandpass filter and either a chirp z-transform or a digital Fourier transform to the at least one cleaned signal to obtain at least one second power spectral density function,
- identifying second spectral peaks in the at least one second power spectral density function,
- evaluating said subject respiratory rate ($R_R$) from said second spectral peaks.

10. System (1) according to claim 8, wherein the measurement unit (3) comprises at least one accelerometer (31) and at least one gyroscope (32), and wherein the at least one first spectral density function comprises a first accelerometer power spectral density function (DSPA1) originating from a measuring signal delivered by the at least one accelerometer (31) and a first gyroscope power spectral density function (DSPG1) originating from measuring signal delivered by the at least one gyroscope (32).

11. System (1) according to claim 10, wherein:

- identifying first spectral peaks comprises identifying accelerometer spectral peaks in the first accelerometer power spectral density function (DSPA1) and gyroscope spectral peaks in the first gyroscope power spectral density function (DSPG1),
- evaluating said subject heart rate ($R_H$) comprises comparing a first relative band power of one chosen accelerometer spectral peak with a second relative band power of one chosen gyroscope peak.

12. System (1) according to claim 7, wherein the measurement unit (3) comprises at least one accelerometer (31) and at least one gyroscope (32), said at least one accelerometer (31) being configured to measure temporal acceleration signals $a_x(t)$, $a_y(t)$ and $a_z(t)$ along three orthogonal axes, and said at least one gyroscope (32) being configured to measure temporal angular rotational speed signals $g_x(t)$, $g_y(t)$ and $g_z(t)$ along three orthogonal axes, wherein step b) comprises, after the pre-processing phase, implementing a principal component analysis on the temporal acceleration signals $a_x(t)$, $a_y(t)$ and $a_z(t)$ and on the temporal angular rotational speed signals $g_x(t)$, $g_y(t)$ and $g_z(t)$, in order to evaluating said subject heart rate ($R_H$) and said subject respiratory rate ($R_R$).

13. System (1) according to claim 8, wherein step b) further comprises:

- processing the at least one first power spectral density signal to obtain at least one seismocardiographic signal (SCG),
- processing said at least one seismocardiographic signal (SCG) to obtain at least one windowed aortic valve opening (AO) peak signal,
- extracting local minima and maxima of the at least one windowed aortic valve opening (AO) peak signal,
- annotating said local minima and maxima with fiducial points.

14. System according to claim 9, wherein step b) further comprises:

- identifying respiratory cycles using said subject respiratory rate ($R_R$),
- processing said respiratory cycles to obtain an inhalation volume of said subject, an estimation of a lung volume of said subject, an estimation of a lung capacity of said subject, an estimation of an inhalation phase and an exhalation phase of said subject.

**Patentansprüche**

1. Verfahren zum Bestimmen einer Herz- oder Atmungsaktivität eines Subjekts, umfassend einen Schritt, bei dem, über wenigstens einen Zeitraum, Probemessungen zu wenigstens einer kinematischen Charakteristik oder einer Position eines von dem Subjekt am Kopf getragenen Zubehörteils (2) gemessen und aufgezeichnet werden, und einen Schritt, bei dem, durch eine Verarbeitungseinheit, diese Probemessungen verarbeitet werden, um die Herz- oder Atmungsaktivität zu bestimmen **dadurch gekennzeichnet, dass** der Schritt des Verarbeitens eine Vorverarbeitungsphase mit folgenden Schritten umfasst:

- Konvertieren von Zeitstempeln der Probemessungen in gewählte Zeiteinheiten,
- Downsampling einer Abtastfrequenz des wenigstens einen aufgezeichneten Signals, um wenigstens ein heruntergetastetes Signal zu erhalten,
- Anwenden wenigstens einer Fensterfunktion auf das wenigstens eine heruntergetastete Signal, um wenigstens ein gefenstertes Signal zu erhalten,
- Wählen wenigstens eines ausgewählten gefensterten Signals aus dem wenigstens einen gefensterten Signal,
- Schätzen der Qualität wenigstens eines ausgewählten gefensterten Signals durch Suchen nach Spitzen in dem wenigstens einen ausgewählten gefensterten Signal.

2. Verfahren nach Anspruch **1,** wobei das Zubehörteil (2) mit wenigstens einem Bewegungssensor ausgestattet ist, wie etwa einem Beschleunigungsmesser (31) oder einem Gyroskop (32) zum Messen der kinematischen Charakteristik oder der Position des Kopfes des Subjekts.

3. Verfahren gemäß einem der Ansprüche 1 bis 2, wobei die Verarbeitung in eingebetteter Weise oder auf einem entfernten Host durchgeführt wird.

4. System (1) zum Bestimmen einer Herz- oder Atmungsaktivität eines Subjekts, wobei das System (1) ein Zubehörteil umfasst (2), das dazu ausgelegt ist, von dem Subjekt am Kopf getragen zu werden und das mit einer Messeinheit (3) ausgestattet ist, die wenigstens einen Bewegungssensor umfasst, wie etwa einen Beschleunigungsmesser (31) oder ein Gyroskop (32), und wenigstens ein Messsignal liefert, das wenigstens eine Position oder eine Geschwindigkeit oder eine Drehgeschwindigkeit oder eine Beschleunigung des Zubehörteils (2) meldet, und eine Verarbeitungseinheit (4), die dafür programmiert ist, diese Schritte zu implementieren:

- a) Empfangen und Aufzeichnen des wenigstens einen Messsignals von der Messeinheit (3), um Probemessungen über wenigstens einen Zeitraum zu erhalten,
- b) Verarbeiten der Probemessungen, die wenigstens ein aufgezeichnetes Signal zum Bestimmen der Herz- oder Atmungsaktivität bilden

**dadurch gekennzeichnet, dass** Schritt b) eine Vorverarbeitungsphase mit folgenden Schritten umfasst:

- Konvertieren von Zeitstempeln der Probemessungen in gewählte Zeiteinheiten,
- Downsampling einer Abtastfrequenz des wenigstens einen aufgezeichneten Signals, um wenigstens ein heruntergetastetes Signal zu erhalten,
- Anwenden wenigstens einer Fensterfunktion auf das wenigstens eine heruntergetastete Signal, um wenigstens ein gefenstertes Signal zu erhalten,
- Wählen wenigstens eines ausgewählten gefensterten Signals aus dem wenigstens einen gefensterten Signal,
- Schätzen der Qualität wenigstens eines ausgewählten gefensterten Signals durch Suchen nach Spitzen in dem wenigstens einen ausgewählten gefensterten Signal.

5. System (1) gemäß Anspruch **4,** wobei das Zubehörteil (2) aus Folgendem gewählt wird: einem Brillengestell, einem Brillenaufsatz, einem Brillenclip, einem Brillenhaltergurtband, einem Kopfhörer, einem Ohrhörer, einem Schmuckstück.

6. System (1) gemäß Anspruch 4 oder Anspruch 5**,** wobei die Verarbeitungseinheit (4) ferner dafür programmiert ist, vor dem Aufzeichnen des wenigstens einen Messsignals folgende Schritte zu implementieren:

- Bestimmen, ob Probemessungen von der Messeinheit (3) eine Auslösebedingung erfüllen,
- falls die Auslösebedingung erfüllt ist, Erhöhen einer Abtastrate, einer Abtastdauer und Empfindlichkeit der Messeinheit (3),
- Auslösen der Aufzeichnung des wenigstens einen Messsignals,

und wobei das Aufzeichnen des wenigstens einen Messsignals über einen Zeitschlitz durchgeführt wird, der in dem wenigstens einen Zeitraum enthalten ist, um die Probemessungen zu erhalten, die wenigstens ein aufgezeichnetes Signal bilden.

7. System (1) gemäß einem der Ansprüche 4 bis 6, wobei, wenn Spitzen während des Qualitätsbewertungsschritts gefunden werden, die Vorverarbeitungsphase ferner einen Schritt umfasst, bestehend aus einer Rauschreduzierung in dem wenigstens einen ausgewählten gefensterten Signal, um wenigstens ein bereinigtes Signal zu erhalten.

8. System (1) gemäß Anspruch 7, wobei Schritt b) nach der Vorverarbeitungsphase dies umfasst:

- Anwenden eines ersten Bandpassfilters, einer Hilbert-Transformation, einer Hüllkurvenanalyse und entweder einer Chirp-z-Transformation oder einer numerischen Fourier-Transformation auf das wenigstens eine bereinigte Signal, um wenigstens eine erste Leistungsspektraldichtefunktion zu erhalten,
- Identifizieren erster Spektralspitzen in der wenigstens einen ersten Leistungsspektraldichtefunktion,
- Auswerten der Herzfrequenz ($R_H$) des Subjekts anhand der ersten Spektralspitzen.

9. System (1) gemäß Anspruch 7, wobei Schritt b) nach der Vorverarbeitungsphase dies umfasst:

- Anwenden eines zweiten Bandpassfilters und entweder einer Chirp-z-Transformation oder einer digitalen Fourier-Transformation auf das wenigstens eine bereinigte Signal, um wenigstens eine zweite Leistungsspektraldichtefunktion zu erhalten,
- Identifizieren zweiter Spektralspitzen in der wenigstens einen zweiten Leistungsspektraldichtefunktion,
- Auswerten der Atemfrequenz ($R_H$) des Subjekts anhand der zweiten Spektralspitzen.

**10.** System (1) gemäß Anspruch 8, wobei die Messeinheit (3) wenigstens einen Beschleunigungsmesser (31) und wenigstens ein Gyroskop (32) umfasst und wobei die wenigstens eine erste Spektraldichtefunktion eine erste Beschleunigungsmesser-Leistungsspektraldichtefunktion (DSPA1) umfasst, die von einem Messsignal stammt, welches von dem wenigstens einen Beschleunigungsmesser (31) geliefert wird, und eine erste Gyroskop-Leistungsspektraldichtefunktion (DSPG1), die von einem Messsignal stammt, welches von dem wenigstens einen Gyroskop (32) geliefert wird.

**11.** System (1) gemäß Anspruch 10, wobei:

- das Identifizieren erster Spektralspitzen das Identifizieren von Beschleunigungsmesser-Spektralspitzen in der ersten Beschleunigungsmesser-Leistungsspektraldichtefunktion (DSPA1) und von Gyroskop-Spektralspitzen in der ersten Gyroskop-Leistungsspektraldichtefunktion (DSPG1) umfasst,
- das Auswerten der Herzfrequenz ($R_H$) des Subjekts das Vergleichen einer ersten relativen Bandleistung einer gewählten Beschleunigungsmesser-Spektralspitze mit einer zweiten relativen Bandleistung einer gewählten Gyroskop-Spektralspitze umfasst.

**12.** System (1) gemäß Anspruch 7, wobei die Messeinheit (3) wenigstens einen Beschleunigungsmesser (31) und wenigstens ein Gyroskop (32) umfasst, wobei der Beschleunigungsmesser (31) dazu ausgelegt ist, zeitliche Beschleunigungssignale $a_x(t)$, $a_y(t)$ und $a_z(t)$ entlang von drei orthogonalen Achsen zu messen, und wobei das wenigstens eine Gyroskop (32) dazu ausgelegt ist, zeitliche Winkeldrehgeschwindigkeitssignale $g_x(t)$, $g_y(t)$ und $g_z(t)$ entlang von drei orthogonalen Achsen zu messen, wobei Schritt b) das Implementieren, nach der Vorverarbeitungsphase, einer Hauptkomponentenanalyse für die zeitlichen Beschleunigungssignale $a_x(t)$, $a_y(t)$ und $a_z(t)$ und für die zeitlichen Winkeldrehgeschwindigkeitssignale $g_x(t)$, $g_y(t)$ und $g_z(t)$ umfasst, um die Herzfrequenz ($R_H$) des Subjekts und die Atemfrequenz ($R_R$) des Subjekts auszuwerten.

**13.** System (1) gemäß Anspruch 8, wobei Schritt b) ferner umfasst:

- Verarbeiten des wenigstens einen ersten Leistungsspektraldichtesignals, um wenigstens ein seismokardiografisches Signal (SCG) zu erhalten,
- Verarbeiten des wenigstens einen seismokardiografischen Signals (SCG), um wenigstens ein gefenstertes Artenklappenöffnung (AO)-Spitzensignals zu erhalten,
- Extrahieren lokaler Minima und Maxima des wenigstens einen gefensterten Artenklappenöffnung (AO)-Spitzensignals,
- Annotieren der lokalen Minima und Maxima mit Referenzpunkten.

**14.** System gemäß Anspruch 9, wobei Schritt b) ferner umfasst:

- Identifizieren von Zyklen unter Verwendung der Atemfrequenz ($R_R$) des Subjekts,
- Verarbeiten der Atemzyklen, um ein Inhalationsvolumen des Subjekts, eine Schätzung eines Lungenvolumens des Subjekts, eine Schätzung einer Lungenkapazität des Subjekts, eine Schätzung einer Einatemphase und einer Ausatemphase des Subjekts zu erhalten.

**Revendications**

**1.** Procédé de détermination de l'activité cardiaque ou respiratoire d'un sujet, comprenant une étape de mesure et d'enregistrement, sur au moins une période de temps, de mesures d'échantillon relatives au moins à une caractéristique cinématique ou à une position d'un accessoire (2) porté par la tête du sujet et une étape de traitement par une unité de traitement de ces mesures d'échantillon pour la détermination de ladite activité cardiaque ou respiratoire **caractérisé en ce que** l'étape de traitement comprend une phase de prétraitement avec les étapes suivantes :

- conversion d'horodatages desdites mesures d'échantillon en unités temporelles choisies,
- réduction de la fréquence d'échantillonnage dudit au moins un signal enregistré afin d'obtenir au moins un signal sous-échantillonné,
- application d'au moins une fonction de fenêtre à l'au moins un signal sous-échantillonné pour obtenir au moins un signal fenêtré,
- choix d'au moins un signal fenêtré sélectionné parmi l'au moins un signal fenêtré,
- estimation de la qualité dudit au moins un signal fenêtré sélectionné en recherchant des pics dans ledit au moins un signal fenêtré sélectionné.

2. Procédé selon la revendication 1, ledit accessoire (2) étant pourvu d'au moins un capteur de mouvement, tel qu'un accéléromètre (31) ou un gyroscope (32) pour mesurer ladite caractéristique cinématique ou ladite position de la tête du sujet.

3. Procédé selon l'une quelconque des revendications 1 et 2, ledit traitement étant réalisé de manière embarquée ou sur un hôte distant.

4. Système (1) de détermination de l'activité cardiaque ou respiratoire d'un sujet, ledit système (1) comprenant un accessoire (2) qui est configuré pour être porté par la tête du sujet et muni d'une unité de mesure (3) comprenant au moins un capteur de mouvement, tel qu'un accéléromètre (31) ou un gyroscope (32) et délivrant au moins un signal de mesure rapportant au moins une position ou une vitesse ou une vitesse de rotation ou une accélération de l'accessoire (2) et une unité de traitement (4) programmée pour mettre en œuvre les étapes de :

   - a) réception et enregistrement dudit au moins un signal de mesure en provenance de l'unité de mesure (3) pour obtenir des mesures d'échantillon sur au moins une période de temps,
   - b) traitement desdites mesures d'échantillon formant au moins un signal enregistré pour déterminer ladite activité cardiaque ou respiratoire

   **caractérisé en ce que** l'étape b) comprend une phase de prétraitement avec les étapes suivantes :

   - conversion d'horodatages desdites mesures d'échantillon en unités temporelles choisies,
   - réduction de la fréquence d'échantillonnage dudit au moins un signal enregistré afin d'obtenir au moins un signal sous-échantillonné,
   - application d'au moins une fonction de fenêtre à l'au moins un signal sous-échantillonné pour obtenir au moins un signal fenêtré,
   - choix d'au moins un signal fenêtré sélectionné parmi l'au moins un signal fenêtré,
   - estimation de la qualité dudit au moins un signal fenêtré sélectionné en recherchant des pics dans ledit au moins un signal fenêtré sélectionné.

5. Système (1) selon la revendication **4,** l'accessoire (2) étant choisi parmi les suivants : une monture de lunettes, un complément de lunettes, un clip de lunettes, un cordon de fixation de lunettes, un casque, un écouteur, un bijou.

6. Système (1) selon la revendication 4 ou la revendication 5, l'unité de traitement (4) étant en outre programmée pour mettre en œuvre, avant l'enregistrement dudit au moins un signal de mesure, les étapes suivantes :

   - détermination si les mesures d'échantillon provenant de l'unité de mesure (3) satisfont à une condition de déclenchement,
   - dans le cas où la condition de déclenchement est remplie, augmentation d'un taux d'échantillonnage, d'une durée d'échantillonnage et de la sensibilité de l'unité de mesure (3),
   - déclenchement de l'enregistrement dudit au moins un signal de mesure,

   et l'enregistrement dudit au moins un signal de mesure étant effectué sur un créneau temporel inclus dans l'au moins une période de temps pour obtenir lesdites mesures d'échantillon formant au moins un signal enregistré.

7. Système (1) selon l'une quelconque des revendications 4 à **6,** lorsque des pics sont trouvés lors de l'étape **d'évaluation** de la qualité, ladite phase de prétraitement comprenant en outre une étape de réduction du bruit dans l'au moins un signal fenêtré sélectionné, pour obtenir au moins un signal nettoyé.

8. Système (1) selon la revendication 7, l'étape b) comprenant, après la phase de prétraitement :

   - l'application d'un premier filtre passe-bande, d'une transformée de Hilbert, d'une analyse d'enveloppe et d'une transformée de compression d'impulsion z ou d'une transformée de Fourier numérique à l'au moins un signal nettoyé pour obtenir au moins une première fonction de densité spectrale de puissance,
   - l'identification de premiers pics spectraux dans l'au moins une première fonction de densité spectrale de puissance,
   - l'évaluation de ladite fréquence cardiaque ($R_H$) du sujet à partir desdits premiers pics spectraux.

**9.** Système (1) selon la revendication 7, l'étape b) comprenant, après la phase de prétraitement :

- l'application d'un deuxième filtre passe-bande et d'une transformée de compression d'impulsion z ou d'une transformée de Fourier numérique à l'au moins un signal nettoyé pour obtenir au moins une deuxième fonction de densité spectrale de puissance,
- l'identification de deuxièmes pics spectraux dans l'au moins une deuxième fonction de densité spectrale de puissance,
- l'évaluation de ladite fréquence respiratoire du sujet ($R_R$) à partir desdits deuxièmes pics spectraux.

**10.** Système (1) selon la revendication 8, l'unité de mesure (3) comprenant au moins un accéléromètre (31) et au moins un gyroscope (32), et au moins une première fonction de densité spectrale comprenant une première fonction de densité spectrale de puissance d'accéléromètre (DSPA1) provenant d'un signal de mesure délivré par au moins un accéléromètre (31) et une première fonction de densité spectrale de puissance de gyroscope (DSPG1) provenant d'un signal de mesure délivré par au moins un gyroscope (32).

**11.** Système (1) selon la revendication 10,

- l'identification des premiers pics spectraux comprenant l'identification des pics spectraux d'accéléromètre dans la première fonction de densité spectrale de puissance d'accéléromètre (DSPA1) et des pics spectraux de gyroscope dans la première fonction de densité spectrale de puissance de gyroscope (DSPG1),
- l'évaluation de ladite fréquence cardiaque ($R_H$) du sujet comprenant la comparaison d'une première puissance de bande relative d'un pic spectral d'accéléromètre choisi à une deuxième puissance de bande relative d'un pic de gyroscope choisi.

**12.** Système (1) selon la revendication 7, l'unité de mesure (3) comprenant au moins un accéléromètre (31) et au moins un gyroscope (32), ledit au moins un accéléromètre (31) étant configuré pour mesurer les signaux d'accélération temporels $a_x(t)$, $a_y(t)$ et $a_z(t)$ le long de trois axes orthogonaux, et ledit au moins un gyroscope (32) étant configuré pour mesurer les signaux de vitesse de rotation angulaire temporelle $g_x(t)$, $g_y(t)$ et $g_z(t)$ le long de trois axes orthogonaux, l'étape b) comprenant, après la phase de prétraitement, la réalisation d'une analyse en composantes principales sur les signaux d'accélération temporels $a_x(t)$, $a_y(t)$ et $a_z(t)$ et sur les signaux de vitesse de rotation angulaire temporelle $g_x(t)$, $g_y(t)$ et $g_z(t)$, afin d'évaluer ladite fréquence cardiaque du sujet ($R_H$) et ladite fréquence respiratoire du sujet ($R_R$).

**13.** Système (1) selon la revendication 8, l'étape b) comprenant en outre :

- le traitement de l'au moins un premier signal de densité spectrale de puissance pour obtenir au moins un signal sismocardiographique (SCG),
- le traitement dudit au moins un signal sismocardiographique (SCG) pour obtenir au moins un signal de pic d'ouverture de valve aortique (AO) fenêtré,
- l'extraction des minima et maxima locaux d'au moins un signal de crête d'ouverture de valve aortique (AO) fenêtré,
- l'annotation desdits minima et maxima locaux avec des points de repère.

**14.** Système selon la revendication 9, l'étape b) comprenant en outre :

- l'identification de cycles respiratoires à l'aide de ladite fréquence respiratoire ($R_R$) du sujet,
- le traitement desdits cycles respiratoires pour obtenir un volume d'inspiration dudit sujet, une estimation d'un volume pulmonaire dudit sujet, une estimation d'une capacité pulmonaire dudit sujet, une estimation d'une phase d'inspiration et d'une phase d'expiration dudit sujet.

## Fig.1

○ R peak

◁ Mitral valve closing    △ Aortic valve opening

▽ Isovolumic moment    ▷ Aortic valve closing

Time (ms)

## Fig.2

## Fig.3

# Fig.4

— Original

# Fig.5

# Fig.6

# Fig.7

# Fig.8

# Fig.9

# Fig.10

R²=0.9658

# Fig.11

# Fig.12

# Fig.13

# Fig.14

**Fig.15**

Xdet

**Fig.16**

Xdet
XdetThre

**Fig.17**

Xdet
XdetThre

EP 4 101 377 B1

# Fig.18

EP 4 101 377 B1

## Fig.19

## Fig.20

## Fig.21

## Fig.22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020037391 A

- US 2015265161 A1